# EUROPEAN PATENT APPLICATION

(11) **EP 4 435 108 A1**
(43) Date of publication of application: **25.09.2024**
(21) Application number: 22911688.4
(22) Date of filing: 06.12.2022
(51) Int. Cl.: C12N 15/77, C12N 9/06, C12P 13/06

(54) **L-ISOLEUCINE-PRODUCING MICROORGANISM AND L-ISOLEUCINE PRODUCING METHOD USING SAME**

(30) Priority: 21.12.2021 KR 20210184151
(71) Applicant: CJ CheilJedang Corporation, Seoul 04560 (KR)
(72) Inventor: KIM, Heejung, Seoul 04560 (KR); CHEONG, Ki Yong, Seoul 04560 (KR); KIM, Heeyeong, Seoul 04560 (KR); CHOI, Woosung, Seoul 04560 (KR)
(74) Representative: Schiweck Weinzierl Koch Patentanwälte Partnerschaft mbB
(86) International application number: PCT/KR2022/019684
(87) International publication number: WO 2023/121055

(57) **Abstract**

The present disclosure relates to a microorganism with L-isoleucine-producing ability, into which a foreign gene encoding glutamate dehydrogenase is introduced, a method of producing L-isoleucine using the microorganism, and a composition for producing L-isoleucine, the composition including the microorganism.

## Description

### [Technical Field]

The present disclosure relates to a microorganism with L-isoleucine-producing ability, into which a foreign gene encoding glutamate dehydrogenase is introduced, a method of producing L-isoleucine using the microorganism, and a composition for producing L-isoleucine, the composition including the microorganism.

### [Background Art]

L-isoleucine is a type of branched-chain amino acids among a total of 20 amino acids, and is classified as an essential amino acid and used in animal feeds, food additives, and pharmaceutical fields. Since L-isoleucine has functions such as post-metabolic energy production, hemoglobin production, blood sugar control, muscle building and repair, etc., its use is increasing not only in fluids, nutrients, and sports nutrition, but also in animal feeds.

For L-isoleucine production, *Corynebacterium glutamicum* and *Escherichia coli* are used as representative microorganisms. In these microorganisms, L-isoleucine shares a major biosynthetic pathway with other branched-chain amino acids, L-valine and L-leucine. With regard to the biosynthetic pathway of L-isoleucine, pyruvate produced during glycolysis and 2-ketobutyrate produced from L-threonine which is an amino acid derived from aspartic acid (aspartate) are used as precursors to finally produce L-isoleucine.

There is known a method of using various microorganisms and variants thereof to produce L-amino acids (US Patent No. 10113190). However, in the method of using variants, many by-products other than L-isoleucine are generated, causing a problem of lowering purity of L-isoleucine in the purification step.

In this regard, to increase the purity of L-isoleucine, a method of purifying L-isoleucine is known (US Patent No. 6072083). However, this purification method has a disadvantage of requiring a separate additional purification process. Thus, there is a need to develop a method of increasing the purity of L-isoleucine.

Meanwhile, L-glutamate is a precursor that provides amine groups in the synthesis of amino acids including L-isoleucine. In producing amino acids, enhanced synthesis of the precursor L-glutamate is essential. However, enhancement of glutamate dehydrogenase, which is known to produce glutamate in L-isoleucine-producing microorganisms, results in a side reaction of producing a byproduct α-aminobutyric acid (AABA) through dehydrogenation of 2-ketobutyrate, which is an intermediate of isoleucine (Microb Cell Fact. 2017 Mar 23;16(1):51). Thus, there is a problem of lowering the L-isoleucine purity and biosynthetic efficiency.

### [Disclosure]

### [Technical Problem]

The present inventors have developed a microorganism with L-isoleucine-producing ability, into which a foreign gene encoding glutamate dehydrogenase is introduced, a method of producing L-isoleucine using the microorganism, and a composition for producing L-isoleucine, the composition including the microorganism, thereby completing the present disclosure.

### [Technical Solution]

An object of the present disclosure is to provide a microorganism with L-isoleucine-producing ability, into which a foreign gene encoding glutamate dehydrogenase derived from *Bacillus subtilis* or *Rhodospirillales* is introduced.

Another object of the present disclosure is to provide a method of producing L-isoleucine, the method including the step of culturing the microorganism in a medium.

Still another object of the present disclosure is to provide a composition for producing L-isoleucine, the composition including the microorganism.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

An aspect of the present disclosure provides a microorganism with L-isoleucine-producing ability, into which a foreign gene encoding glutamate dehydrogenase derived from *Bacillus subtilis* or *Rhodospirillales* is introduced.

As used herein, the term "L-isoleucine", one of the essential amino acids, refers to an L-amino acid with a chemical formula of HO₂CCH(NH₂)CH(CH₃)CH₂CH₃, which structurally corresponds to branched chain amino acids, along with L-valine and L-leucine.

As used herein, the term "strain (or microorganism)" includes all wild-type microorganisms or naturally or artificially genetically modified microorganisms, and it may be a microorganism in which a specific mechanism is weakened or strengthened due to insertion of a foreign gene or activity enhancement or inactivation of an endogenous gene, and it may be a microorganism including genetic modification for production of a desired polypeptide, protein, or product.

As used herein, the term "microorganism with L-isoleucine-producing ability" refers to a microorganism naturally having the L-isoleucine-producing ability or a microorganism obtained by providing the L-isoleucine-producing ability to a parent strain without the L-isoleucine-producing ability. Specifically, the microorganism may be a microorganism producing L-isoleucine, into which a foreign gene encoding glutamate dehydrogenase derived from *Bacillus subtilis* or *Rhodospirillales* is introduced, but is not limited thereto.

Specifically, the "L-isoleucine-producing microorganism" includes all of wild-type microorganisms or naturally or artificially genetically modified microorganisms. More specifically, the microorganism may be a microorganism in which a specific mechanism is weakened or strengthened due to insertion of a foreign gene or activity enhancement or inactivation of an endogenous gene, and it may be a microorganism in which genetic modification occurs or L-isoleucine production activity is enhanced in order to produce the desired L-isoleucine. With respect to the object of the present disclosure, the microorganism with L-isoleucine-producing ability is characterized in that the desired L-isoleucine-producing ability is increased by introducing the foreign gene encoding glutamate dehydrogenase derived from *Bacillus subtilis* or *Rhodospirillales,* and may be a genetically modified microorganism or recombinant microorganism, but is not limited thereto.

As used herein, the term "introduction" of activity means that a gene not originally possessed by a microorganism is expressed within the microorganism to exhibit the activity of a specific protein or to exhibit the increased or improved activity as compared to endogenous activity of the corresponding protein or the activity before modification. For example, a polynucleotide encoding a specific protein may be introduced into a chromosome in a microorganism, or a vector containing the polynucleotide encoding the specific protein may be introduced into the microorganism, thereby exhibiting its activity.

For example, the recombinant strain having the increased production ability may have an increased L-isoleucine-producing ability of about 1% or more, specifically, about 1% or more, about 2% or more, about 3% or more, about 4% or more, about 5% or more, about 6% or more, about 7% or more, about 8% or more, about 8.1% or more, about 8.2% or more, or about 8.3% or more (the upper limit is not particularly limited, but may be, for example, about 100% or less, about 50% or less, about 25% or less, about 20% or less, about 15% or less, or about 10% or less), as compared to that of the parent strain before modification or an unmodified microorganism having endogenous activity of the gdh protein, but the increased amount is not limited thereto as long as the producing ability has an increased amount of a + value, as compared to the production ability of the parent strain before modification or the unmodified microorganism. In another example, the recombinant strain having the increased production ability may have an increased L-isoleucine-producing ability of about 1.01 times or more, about 1.02 times or more, about 1.03 times or more, about 1.04 times or more, about 1.05 times or more, about 1.06 times or more, about 1.07 times or more, or about 1.08 times or more (the upper limit is not particularly limited, but may be, for example, about 10 times or less, about 5 times or less, about 3 times or less, about 2 times or less, about 1.5 times or less, or about 1.1 times or less), as compared to that of the parent strain before modification or the unmodified microorganism, but is not limited thereto.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may refer to a strain before introducing the foreign glutamate dehydrogenase gene described in this specification. The "unmodified microorganism" may be used interchangeably with "strain before modification", "microorganism before modification", "unvaried strain", "unmodified strain", "unvaried microorganism", or "reference microorganism".

In another example of the present disclosure, the microorganism of the present disclosure may be a microorganism capable of producing L-isoleucine, and its type is not particularly limited. The microorganism of the present disclosure may be either a prokaryotic cell or a eukaryotic cell, specifically, a prokaryotic cell. The prokaryotic cell may include, for example, microbial strains of the genus *Corynebacterium,* the genus *Escherichia,* the genus *Erwinia,* the genus *Seratia,* the genus *Providencia,* and the genus *Brevibacterium,* specifically, microorganisms of the genus *Corynebacterium.*

In the present disclosure, the "microorganisms of the genus *Corynebacterium"* may include all microorganisms of the genus *Corynebacterium.* Specifically, it may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* more specifically, *Corynebacterium glutamicum.*

As used herein, the term "glutamate dehydrogenase" is an enzyme that synthesizes glutamate which is a precursor for L-isoleucine biosynthesis. In the present disclosure, the "glutamate dehydrogenase" may be used interchangeably with "gdh", "rocG".

As used herein, the terms "protein having glutamate dehydrogenase activity" and "gene encoding glutamate dehydrogenase" may include any protein having the glutamate dehydrogenase activity as described above and any gene encoding the same without limitation. Specifically, the glutamate dehydrogenase is known in the art, and protein and gene sequences of the glutamate dehydrogenase may be obtained from known databases, and examples thereof may include the NCBI GenBank, etc., but are not limited thereto.

Meanwhile, L-glutamate is a precursor that provides amine groups in the synthesis of amino acids including L-isoleucine. In producing amino acids, enhanced synthesis of the precursor L-glutamate is essential. However, enhancement of glutamate dehydrogenase, which is known to produce glutamate in L-isoleucine-producing microorganisms, results in a side reaction of producing a byproduct α-aminobutyric acid (AABA) through dehydrogenation of 2-ketobutyrate, which is an intermediate of isoleucine (Microb Cell Fact. 2017 Mar 23;16(1):51). Thus, there is a problem of lowering the L-isoleucine purity and biosynthetic efficiency.

In the microorganism with the L-isoleucine-producing ability of the present disclosure, the foreign gene encoding glutamate dehydrogenase derived from *Bacillus subtilis* or *Rhodospirillales* is introduced, thereby reducing by-product generation. The by-product may be α-aminobutyric acid (AABA). The reduction of the by-product generation may mean reduction of α-aminobutyric acid (AABA) production, relative to the L-isoleucine production, as compared to that of the wild-type microorganism, but is not limited thereto.

With respect to the object of the present disclosure, the protein having the glutamate dehydrogenase activity may be derived from *Bacillus subtilis* or *Rhodospirillales.* Specifically, the glutamate dehydrogenase may have and/or may include an amino acid sequence represented by SEQ ID NO: 1 or SEQ ID NO: 3, or may essentially consist of or may consist of the amino acid sequence.

Further, the glutamate dehydrogenase of the present disclosure may include an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1 or SEQ ID NO: 3. It is also apparent that any glutamate dehydrogenase having an amino acid sequence with deletion, modification, substitution, conservative substitution, or addition in part of the sequence may also be included within the scope of the present disclosure as long as the amino acid sequence has such homology or identity and exhibits efficacy corresponding to that of the glutamate dehydrogenase of the present disclosure.

In the present disclosure, although being described as 'a polypeptide or protein including an amino acid sequence represented by a specific SEQ ID NO.', 'a polypeptide or protein consisting of an amino acid sequence represented by a specific SEQ ID NO.', or 'a polypeptide or protein having an amino acid sequence represented by a specific SEQ ID NO.', it is obvious that any protein having an amino acid sequence with deletion, modification, substitution, conservative substitution, or addition of some sequence may be used in the present disclosure as long as the protein has activity identical or corresponding to that of the polypeptide consisting of the amino acid sequence of the corresponding SEQ ID NO. For example, there are cases of having the addition of a sequence which does not alter the function of the protein at the N-terminus and/or C-terminus of the amino acid sequence, a naturally occurring mutation, a silent mutation thereof, or a conservative substitution.

For example, there are cases of having the addition or deletion of a sequence which does not alter the function of the glutamate dehydrogenase of the present disclosure at the N-terminus, C-terminus, and/or inside the amino acid sequence, a naturally occurring mutation, a silent mutation thereof, or a conservative substitution.

As used herein, the term "conservative substitution" means substituting an amino acid with another amino acid having similar structural and/or chemical properties. Such amino acid substitution may generally occur based on similarity in the polarity, charge, solubility, hydrophobicity, hydrophilicity and/or amphipathic nature of the residue. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Further, amino acids may be classified into amino acids with electrically charged side chains and amino acids with uncharged side chains. The amino acids with electrically charged side chains include aspartic acid, glutamic acid, lysine, arginine, and histidine, and the amino acids with uncharged side chains may be further classified into nonpolar amino acids or polar amino acids. The nonpolar amino acids include glycine, alanine, valine, leucine, isoleucine, methionine, phenylalanine, tryptophan, and proline, and polar amino acids include serine, threonine, cysteine, tyrosine, asparagine, and glutamine. Commonly, the conservative substitution may have little or no effect on the activity of the produced polypeptide. Commonly, the conservative substitution may have little or no effect on the activity of the protein or polypeptide.

Further, the glutamate dehydrogenase may also include deletion or addition of amino acids that have minimal influence on the properties and secondary structure of the polypeptide. For example, a polypeptide may be conjugated to a signal (or leader) sequence at the N-terminus of the protein, which co-translationally or post-translationally directs transfer of the protein. The polypeptide may also be conjugated to other sequence or a linker for identification, purification, or synthesis of the polypeptide.

As used herein, the term 'homology' or 'identity' refers to a degree of similarity between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program used may be used together. Substantially, homologous or identical sequences may generally hybridize under moderately or highly stringent conditions to the entirety or a part of the sequence. It is apparent that hybridization also includes hybridization of a polynucleotide with a polynucleotide including a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package ((Devereux, J., et al, Nucleic Acids Research 12: 387(1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al.(1970), J Mol Biol. 48:443, as disclosed by Smith and Waterman, Adv. Appl. Math(1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358(1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

As used herein, the term "corresponding to" refers to an amino acid residue at a position listed in a polypeptide, or an amino acid residue similar to, identical to, or homologous to a residue listed in a polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the numerical position of the amino acid residue corresponding to the amino acid residue of SEQ ID NO: 1. For example, a sequence alignment algorithm as described in the present disclosure may determine the position of an amino acid, or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto, and a sequence alignment program, a pairwise sequence comparison algorithm, etc., which known in the art, may be appropriately used.

As used herein, the term "endogenous activity" refers to activity at a native state originally possessed by a microorganism or activity of the protein possessed before modification of the corresponding protein. This may be used interchangeably with "activity before modification".

As used herein, the term "enhancement" of the polypeptide activity means that the activity of the polypeptide is increased, as compared to the endogenous activity thereof. The enhancement may be used interchangeably with terms such as activation, up-regulation, overexpression, and increase, etc. Here, the activation, enhancement, up-regulation, overexpression, and increase may include both cases in which an activity not originally possessed is exhibited, or the activity is enhanced, as compared to the endogenous activity or the activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased, as compared to the endogenous activity means that the activity of the polypeptide is improved as compared to the activity and/or concentration (expression level) of the specific polypeptide originally possessed by a parent strain before the trait is changed or an unmodified microorganism.

The enhancement may be achieved through the introduction of a foreign polypeptide or the enhancement of the activity of the endogenous polypeptide, and concentration (expression level). The enhancement of the activity of the polypeptide may be confirmed by an increase in the degree of activity and the expression level of the corresponding polypeptide or increase in the amount of the product released from the corresponding polypeptide.

For the enhancement of the activity of the polypeptide, various methods well known in the art may be applied, and the method is not limited as long as the activity of the desired polypeptide may be enhanced as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) modification of a gene expression regulatory region on a chromosome encoding the polypeptide (e.g., occurrence of variations in the expression regulatory region, replacement with a sequence having stronger activity, or insertion of a sequence having stronger activity);
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically, 1) the increase in the intracellular copy number of the polynucleotide encoding the polypeptide may be achieved by introducing, into a host cell, a vector which may replicate and function independently of the host and to which the polynucleotide encoding the corresponding polypeptide is operably linked. Alternatively, the increase may be achieved by introducing one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.

2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may include, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.

Examples of known strong promoters include CJ1 to CJ7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, an yccA promoter, etc., but is not limited thereto.

3) The modification of a nucleotide sequence encoding an initiation codon or 5'-UTR region of the gene transcript encoding the polypeptide may be, for example, substitution with another initiation codon having a higher polypeptide expression rate as compared to an endogenous initiation codon, but is not limited thereto.

4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion. The selection marker is as described above.

6) The introduction of a foreign polynucleotide exhibiting the activity of the polypeptide may be introduction of a foreign polynucleotide into a host cell, the foreign polynucleotide encoding a polypeptide exhibiting activity identical or similar to that of the polypeptide. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in the host cell, the polypeptide may be produced and the activity thereof may be increased.

7) The codon optimization of the polynucleotide encoding the polypeptide may be codon optimization of an endogenous polynucleotide so as to enhance transcription or translation in a host cell, or codon optimization of a foreign polynucleotide so as to perform optimized transcription and translation in a host cell.

8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration/expression level of the corresponding polypeptide, based on the activity or concentration of the polypeptide expressed in a wild-type or a microbial strain before being modified, or an increase in the amount of a product produced from the corresponding polypeptide, but is not limited thereto.

Specifically, with respect to the object of the present disclosure, the microorganism may be introduced with a foreign polynucleotide having glutamate dehydrogenase activity, which is derived from *Bacillus subtilis* or *Rhodospirillales,* so as to enhance the activity of glutamate dehydrogenase protein.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant.

The polynucleotide according to the present disclosure is characterized by being derived from *Bacillus subtilis* or *Rhodospirillales.* Specifically, the polynucleotide may include a nucleotide sequence encoding an amino acid sequence of SEQ ID NO: 1 or SEQ ID NO: 3. More specifically, the foreign gene encoding glutamate dehydrogenase derived from *Bacillus subtilis* and the foreign gene encoding glutamate dehydrogenase derived from *Rhodospirillales* may have nucleotide sequences of SEQ ID NO: 2 and SEQ ID NO: 4, respectively, but are not limited thereto. The nucleotide sequences of SEQ ID NO: 2 and SEQ ID NO: 4 may be obtained from a known database, and examples thereof may be NCBI GenBank, etc., but is not limited thereto.

In the present disclosure, the gene including the nucleotide sequence of SEQ ID NO: 2 or SEQ ID NO: 4 may be used interchangeably with a polynucleotide including the nucleotide sequence of SEQ ID NO: 2 or SEQ ID NO: 4, a gene or polynucleotide having the nucleotide sequence of SEQ ID NO: 2 or SEQ ID NO: 4, or a gene or polynucleotide consisting of the nucleotide sequence of SEQ ID NO: 2 or SEQ ID NO: 4.

The polynucleotide of the present disclosure may undergo various modifications in the coding region without changing the amino acid sequence of the variant of the present disclosure, due to codon degeneracy or in consideration of the codons preferred in an organism in which the variant of the present disclosure is to be expressed. Specifically, the polynucleotide of the present disclosure may have or may include a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2 or SEQ ID NO: 4, or may consist of or may essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2 or SEQ ID NO: 4, but is not limited thereto.

Further, the polynucleotide of the present disclosure may include a probe that may be prepared from a known gene sequence, for example, may include any sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, i.e., polynucleotides having 70% or more, 75% or more, 6% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

With respect to the object of the present disclosure, the microorganism includes an expression vector for expressing the foreign polynucleotide in a host, thereby having the enhanced glutamate dehydrogenase protein activity, as compared to the endogenous activity, but is not limited thereto.

The vector of the present disclosure may include a DNA construct including a base sequence of a polynucleotide encoding a desired polypeptide which is operably linked to a suitable expression regulatory region (or expression control sequence) so that the desired polypeptide may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pSK system, pSKH system, pET system, etc. may be used as a plasmid vector. Specifically, pDZ, pDC, pDCM2, pACYC177, pACYC184, pCL, pSK, pSKH130, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide includes DNA and/or RNA encoding a desired polypeptide. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the desired variant of the present disclosure.

The modification of a part or the entirety of the polynucleotide in the microorganism of the present disclosure may be induced by: (a) homologous recombination using a vector for chromosomal insertion in a microorganism or genome editing using engineered nuclease (e.g., CRISPR-Cas9), or and/or (b) the treatment with light, such as ultraviolet light and radiation, and/or chemicals, but is not limited thereto. The method of modifying a part or the entirety of the gene may include a method by DNA recombinant technology. For example, a nucleotide sequence or vector containing a nucleotide sequence homologous to a target gene may be introduced into the microorganism to bring about homologous recombination, resulting in deletion in a part or the entirety of the gene. The nucleotide sequence or vector to be introduced may include a dominant selection marker, but is not limited thereto.

Meanwhile, in a specific embodiment of the present disclosure, the microorganism of the genus *Corynebacterium* may further include an enzyme with enhanced activity, which is involved in the L-isoleucine biosynthetic pathway.

As used herein, the term "enzyme involved in the L-isoleucine biosynthetic pathway" may include aspartate kinase (lysC gene), aspartate-β-semialdehyde dehydrogenase (asd gene), homoserine dehydrogenase (hom gene), homoserine kinase (thrB gene), threonine synthase (thrC gene), threonine dehydratase (ilvA gene), aminotransferase (ilvE gene), etc., but is not limited thereto.

Another aspect of the present disclosure provides a method of producing L-isoleucine, the method including the step of culturing the microorganism in a medium.

The microorganism and L-isoleucine are as described above.

Specifically, the foreign gene encoding glutamate dehydrogenase derived from *Bacillus subtilis;* and the foreign gene encoding glutamate dehydrogenase derived from *Rhodospirillales* may have nucleotide sequences of SEQ ID NOS: 2 and 4, respectively, but are not limited thereto.

Specifically, the method may reduce the production amount of α-aminobutyric acid (AABA), relative to the production amount of L-isoleucine, but is not limited thereto.

As used herein, the term "culturing" refers to growing the microorganism of the present disclosure in appropriately adjusted environment conditions. The culture procedure of the present disclosure may be performed according to appropriate media or culture conditions known in the art. Such culture may be easily adjusted according to the selected strain by a person skilled in the art. Specifically, the culture may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials including water essential for survival and growth, growth factors, etc. Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, etc.

In the present disclosure, the carbon source may include carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may include potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be included. These constituent ingredients or precursors may be added to the medium in a batch or continuous manner. However, the present disclosure is not limited thereto.

The pH of the culture may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

The culture temperature may be maintained at 25°C to 40°C, more specifically, at 28°C to 37°C, but is not limited thereto. The culturing may be continued until the desired amount of useful substances is produced, specifically, for 1 hour to 100 hours, but is not limited thereto.

The L-isoleucine produced by the culturing of the present disclosure may be released into the medium or may remain in cells.

The method of producing a L-isoleucine of the present disclosure may further include the steps of preparing the microorganism of the present disclosure, preparing a medium for culturing the microorganism, or a combination thereof (regardless of the order, in any order), for example, before or after the culturing step.

The method of producing L-isoleucine of the present disclosure may further include the step of recovering the L-isoleucine from a medium resulting from the culturing (a medium in which culturing has been performed) or the microorganism. The recovering step may be further included after the culturing step.

The recovering may be collecting the desired L-isoleucine by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and the desired L-isoleucine may be recovered from the medium or microorganism by using an appropriate method known in the art.

In addition, the method of producing L-isoleucine of the present disclosure may further include a purification step. The purification may be performed by using an appropriate method known in the art. In an exemplary embodiment, when the method of producing L-isoleucine of the present disclosure includes both the recovering step and the purification step, the recovering step and the purification step may be performed continuously or discontinuously regardless of the order, or may be performed simultaneously or integrated into one step, but is not limited thereto.

Still another aspect of the present disclosure provides a composition for producing L-isoleucine, the composition including the microorganism.

The microorganism and L-isoleucine are as described above.

Specifically, the foreign gene encoding glutamate dehydrogenase derived from *Bacillus subtilis;* and the foreign gene encoding glutamate dehydrogenase derived from *Rhodospirillales* may have nucleotide sequences of SEQ ID NOS: 2 and 4, respectively, but are not limited thereto.

Specifically, the method may reduce the production amount of α-aminobutyric acid (AABA), relative to the production amount of L-isoleucine, but is not limited thereto.

The composition of the present disclosure may further include any appropriate excipient that is usually used in the composition for producing L-isoleucine, and such excipients may include, for example, a preserving agent, a wetting agent, a dispersing agent, a suspending agent, a buffering agent, a stabilizing agent, an isotonic agent, etc., but are not limited thereto.

Still another aspect of the present disclosure provides use of the microorganism with the L-isoleucine-producing ability of the present disclosure, into which the foreign gene encoding glutamate dehydrogenase derived from *Bacillus subtilis* or *Rhodospirillales* is introduced, for producing L-isoleucine.

### [Advantageous Effects]

A microorganism with L-isoleucine-producing ability of the present disclosure, into which a foreign gene encoding glutamate dehydrogenase is introduced, may produce L-isoleucine in a high yield by producing less by-products, and accordingly, it may be usefully applied to the industrial production of L-isoleucine.

### [Mode for Carrying Out the Invention]

Hereinafter, the present disclosure will be described in more detail with reference to exemplary embodiments. However, these exemplary embodiments are only for illustrating the present disclosure, and the scope of the present disclosure is not intended to be limited by these exemplary embodiments.

### Example 1: Construction of Recombinant Vector for Introducing Foreign Glutamate Dehydrogenase

To insert a foreign gdh-overexpressing vector into the chromosome of *Corynebacterium glutamicum,* NCgl2872, which is known as a gene encoding a transposon in *Corynebacterium glutamicum,* was used as an insertion site (Journal of Biotechnology 104, 5-25 Jorn Kalinowski et al, 2003). To replace the NCgl2872 gene with a foreign gdh, a vector for NCgl2872 deletion and target gene insertion was constructed. To construct the vector, PCR was performed using the chromosome of ATCC13032 as a template and primer pairs of SEQ ID NO: 5, SEQ ID NO: 6, SEQ ID NO: 7, and SEQ ID NO: 8, respectively. PfuUltra^{™} high-fidelity DNA polymerase (Stratagene) was used as a polymerase for PCR reaction, and PCR conditions included denaturation at 95°C for 30 seconds; annealing at 55°C for 30 seconds; and polymerization at 72°C for 1 minute, and denaturation, annealing, and polymerization under these conditions were repeated 28 times. As a result, DNA fragments of 623 bp and 620 bp were obtained, respectively. The obtained DNA products were purified using a PCR purification kit (QUIAGEN), and cloned with a heat-treated pDCM2 vector (Korean Patent Publication No. 10-2020-0136813) using an infusion cloning kit (TaKaRa) according to the provided manual, thereby constructing a vector pDCM2ΔN2872 for NCgl2872 deletion and target gene insertion.

To produce a strain having the gdh promoter of the parent strain *Corynebacterium glutamicum,* into which a foreign gdh was introduced, PCR was performed using each of the chromosomes of *Corynebacterium glutamicum* ATC13032, *E*. *coli, Bacillus subtilis, Rhodospirillales,* and *Mycobacterium smegmatis* as a template and primers of SEQ ID NO: 9 and SEQ ID NO: 10; or SEQ ID NO: 9 and SEQ ID NO: 11; or SEQ ID NO: 12 and SEQ ID NO: 13; or SEQ ID NO: 14 and SEQ ID NO: 15; or SEQ ID NO: 16 and SEQ ID NO: 17; or SEQ ID NO: 18 and SEQ ID NO: 19. Sequences of the primers used to perform each PCR are shown in Table 1 below.

**[Table 1]**

| SEQ ID NO. | Name | Sequence |
|---|---|---|
| 5 | primer | tgaattcgagctcggtacccAGGCGCAGGGCCG |
| | | GG |
| 6 | primer | gggCAACGCCCACACGCAGCG |
| 7 | primer | AgggGCCCCGCTGAAGTCATC |
| 8 | primer | gactctagaggatccccGCAGATCCAGTCCATC CC |
| 9 | primer | CTGCGTGTGGGCGTTGcccTACCAATTCC ATTTGAGGGC |
| 10 | primer | GATTTCCTCGTTCCCA |
| 11 | primer | ATGACTTCAGCGGGGCcccTTAGATGACG CCCTGTGCCA |
| 12 | primer | GATGGGAACGAGGAAATCATGGATCAGA CATATTCTCT |
| 13 | primer | GATGACTTCAGCGGGGCcccTTAAATCAC ACCCTGCGCC |
| 14 | primer | GATGGGAACGAGGAAATCATGTCAGCAA AGCAAGTCTC |
| 15 | primer | GATGACTTCAGCGGGGCcccTTAGACCCA TCCGCGGAA |
| 16 | primer | GATGGGAACGAGGAAATCATGTCTAAAA GCCACAGCGG |
| 17 | primer | GATGACTTCAGCGGGGCcccTTAAACCAC ACCTTCCGC |
| 18 | primer | GATGGGAACGAGGAAATCATGAGCGAAC TACACCCCAA |
| 19 | primer | GATGACTTCAGCGGGGCcccTCAGATGAG CCCGAGTGCG |

PfuUltra^{™} high-fidelity DNA polymerase (Stratagene) was used as a polymerase for the PCR reaction, and PCR conditions included denaturation at 95°C for 30 seconds; annealing at 55°C for 30 seconds; and polymerization at 72°C for 1 minute, and denaturation, annealing, and polymerization under these conditions were repeated 28 times. As a result, a 519 bp DNA fragment of the gdh promoter region, an 1882 bp DNA fragment of the *Corynebacterium glutamicum* ATCC13032 gdh region including the promoter, a 1382 bp DNA fragment of the *E.coli* gdh region, a 1313 bp DNA fragment of the *Bacillus subtilis* gdh (rocG) region, a 1424 bp DNA fragment of the *Rhodospirillales* gdh region, and a 1388 bp DNA fragment of the *Mychobacterium smegmatis* gdh region were obtained, respectively.

PCR was performed using the amplified promoter and foreign gdh DNA fragments as templates and primers of SEQ ID NO: 9 and SEQ ID NO: 13; or SEQ ID NO: 9 and SEQ ID NO: 15; or SEQ ID NO: 9 and SEQ ID NO: 17; or SEQ ID NO: 9 and SEQ ID NO: 19, respectively. PCR conditions included denaturation at 95°C for 5 minutes, followed by 28 cycles of denaturation at 95°C for 30 seconds; annealing at 55°C for 30 seconds; and polymerization at 72°C for 2 minutes, and then polymerization at 72°C for 5 minutes.

As a result, 2 Kb foreign gdh DNA fragment having the *Corynebacterium glutamicum* ATCC13032 gdh promoter and encoding the foreign glutamate dehydrogenase was amplified. The amplification product was purified using a PCR purification kit (QUIAGEN), which was used as an insert DNA fragment for vector construction. The purified amplification product was treated with restriction enzyme Smal, and then pDCM2ΔN2872 vector heat-treated at 65°C for 20 minutes and the amplification product insert DNA fragment were prepared at a molar concentration ratio (M) of 1:2, and cloned using an infusion cloning kit (TaKaRa) according to the provided manual, thereby constructing vectors for introducing the foreign gdh into chromosome, pDCM2ΔN2872::Pn_gdh (c.gl), pDCM2ΔN2872::Pn_gdh (E.coli), pDCM2ΔN2872::Pn_rocG (B.su), pDCM2ΔN2872::Pn_gdh (rhodospirillales), and pDCM2ΔN2872::Pn_gdh (m.sm).

### Example 2: Preparation of Strain of Genus Corynebacterium having L-isoleucine-producing ability

Wild-type *Corynebacterium glutamicum* has the ability to produce L-isoleucine, but does not produce L-isoleucine in excessive amounts. Accordingly, in order to identify genetic traits that increase the L-isoleucine-producing ability according to the object of the present disclosure, it was attempted to utilize a strain with the increased L-isoleucine-producing ability.

First, an L-isoleucine-producing strain was developed from wild-type *Corynebacterium glutamicum* ATCC13032. In detail, to release the feedback inhibition of threonine, which is a precursor of isoleucine, in the L-isoleucine biosynthetic pathway, the gene horn encoding homoserine dehydrogenase was mutated to substitute arginine, which is an amino acid at position 407 of homoserine dehydrogenase, with histidine (Korean Patent No. 10-1996769). Specifically, a polynucleotide sequence encoding hom(R407H) is represented by SEQ ID NO: 20.

In detail, to prepare strains into which the hom(R407H) mutation was introduced, PCR was performed using the chromosome of *Corynebacterium glutamicum* ATCC13032 as a template and primers of SEQ ID NO: 21 and SEQ ID NO: 22; or SEQ ID NO: 23 and SEQ ID NO: 24, respectively. Sequences of the primers used to perform each of the above PCRs are shown in Table 2 below.

**[Table 2]**

| SEQ ID NO. | Name | Sequence |
|---|---|---|
| 21 | primer | TCGAGCTCGGTACCCCGCTTTTGCACT CATCGAGC |
| 22 | primer | CACGATCAGATGTGCATCATCAT |
| 23 | primer | ATGATGATGCACATCTGATCGTG |
| 24 | primer | CTCTAGAGGATCCCCGAGCATCTTCCA AAACCTTG |

PfuUltra^{™} high-fidelity DNA polymerase (Stratagene) was used as a polymerase for the PCR reaction, and PCR conditions included denaturation at 95°C for 30 seconds; annealing at 55°C 30 seconds; and polymerization at 72°C for 1 minute, and the denaturation, annealing, and polymerization reactions under these conditions were repeated 28 times. As a result, centering the mutation of the horn gene, a 1000 bp DNA fragment of the 5' upstream region and a 1000 bp DNA fragment of the 3' downstream region were obtained.

PCR was performed using the two amplified DNA fragments as templates and primers of SEQ ID NO: 21 and SEQ ID NO: 24. PCR conditions included denaturation at 95°C for 5 minutes, followed by 28 cycles of denaturation at 95°C for 30 seconds; annealing at 55°C 30 seconds; and polymerization at 72°C for 2 minutes, and then polymerization at 72°C for 5 minutes.

As a result, a 2 kb DNA fragment was amplified, which included a mutation in the horn gene encoding the homoserine dehydrogenase variant in which arginine at position 407 was substituted with histidine. The amplification product was purified using a PCR purification kit (QUIAGEN) and used as an insert DNA fragment for vector construction. The purified amplification product was treated with restriction enzyme Smal, and then pDCM2 vector heat-treated at 65°C for 20 minutes and the amplification product insert DNA fragment were prepared at a molar concentration ratio (M) of 1:2, and cloned using an infusion cloning kit (TaKaRa) according to the provided manual, thereby constructing a vector pDCM2-R407H for introducing the hom(R407H) mutation into chromosome.

The constructed vector was transformed into *Corynebacterium glutamicum* ATCC13032 by electroporation, and through a second crossover process, a strain containing the hom(R407H) mutation on the chromosome was obtained, which was named *Corynebacterium glutamicum* ATCC13032 hom(R407H).

In order to release feedback on L-isoleucine and to increase the activity in the prepared ATCC13032 hom(R407H) strain, ilvA, which is a gene encoding L-threonine dehydratase was mutated such that threonine which is an amino acid at position 381 in L-threonine dehydratase (SEQ ID NO: 25) was substituted with alanine and phenylalanine which is an amino acid at position 383 was substituted with alanine. Further, a strain into which ilvA (T381A+F383A) was introduced was prepared.

In detail, to prepare strains into which the ilvA (T381A+F383A) mutation was introduced, PCR was performed using the chromosome of *Corynebacterium glutamicum* ATCC13032 as a template and primers of SEQ ID NO: 26 and SEQ ID NO: 27; or SEQ ID NO: 28 and SEQ ID NO: 29, respectively. Sequences of the primers used to perform each of the above PCRs are shown in Table 3 below.

**[Table 3]**

| SEQ ID NO. | Name | Sequence |
|---|---|---|
| 26 | primer | TCGAGCTCGGTACCCATGAGTGAAACATACG TGTC |
| 27 | primer | GCGCTTGAGGTACTCtgcCAGCGcGATGTCAT CATCCGG |
| 28 | primer | CCGGATGATGACATCgCGCTGgcaGAGTACC TCAAGCGC |
| 29 | primer | CTCTAGAGGATCCCCCGTCACCGACACCTC CACA |

PfuUltra^{™} high-fidelity DNA polymerase (Stratagene) was used as a polymerase for the PCR reaction, and PCR conditions included denaturation at 95°C for 30 seconds; annealing at 55°C 30 seconds; and polymerization at 72°C for 1 minute, and the denaturation, annealing, and polymerization reactions under these conditions were repeated 28 times. As a result, centering the mutation of the ilvA gene, a 1126 bp DNA fragment of the 5' upstream region and a 286 bp DNA fragment of the 3' downstream region were obtained.

PCR was performed using the two amplified DNA fragments as templates and primers of SEQ ID NO: 26 and SEQ ID NO: 29. PCR conditions included denaturation at 95°C for 5 minutes, followed by 28 cycles of denaturation at 95°C for 30 seconds; annealing at 55°C 30 seconds; and polymerization at 72°C for 2 minutes, and then polymerization at 72°C for 5 minutes.

As a result, a 1.4 kb DNA fragment was amplified, which included a mutation in the ilvA gene encoding the L-threonine dehydratase variant in which threonine at position 381 was substituted with alanine and phenylalanine at position 383 was substituted with alanine. The amplification product was purified using a PCR purification kit (QUIAGEN) and used as an insert DNA fragment for vector construction. The purified amplification product was treated with restriction enzyme Smal, and then pDCM2 vector heat-treated at 65°C for 20 minutes and the amplification product insert DNA fragment were prepared at a molar concentration ratio (M) of 1:2, and cloned using an infusion cloning kit (TaKaRa) according to the provided manual, thereby constructing a vector pDCM2-ilvA(T381A+F383A) for introducing the ilvA(T381A+F383A) mutation into chromosome.

The constructed vector was transformed into *Corynebacterium glutamicum* ATCC13032 hom(R407H) by electroporation, and through a second crossover process, a strain containing the ilvA(T381A+F383A) mutation on the chromosome was obtained, which was named *Corynebacterium glutamicum* CA10-3101.

For reference, to confirm whether or not the introduction of ilvA (T381A+F383A) into the L-isoleucine producing strain increases the L-isoleucine production efficiency by releasing feedback on L-isoleucine and by increasing activity, the following experiment was performed. In detail, the results of measuring the L-isoleucine and L-threonine concentrations in the culture medium of the KCCM11248P/pECCG117-ilvA(T381A+F383A) strain, in which the ilvA(T381A+F383A) mutation was introduced by an electric pulse method into KCJI-38 (KCCM11248P, Korean Patent No. 10-1335789) strain which is an L-isoleucine producing strain treated with N-methyl-N'-nitro-N-nitrosoguanidine (NTG), are shown in Table 4 below.

**[Table 4]**

| Name of strain | L-isoleucine (g/L) | L-threonine (g/L) |
|---|---|---|
| KCCM11248P | 1.5 | 0.5 |
| KCCM11248P/pECCG117-ilvA(F383A) | 2.8 | 0.6 |
| KCCM11248P/pECCG117-ilvA(T381A+F383A) | 4.0 | 0.0 |

As shown in Table 3, the KCCM11248P/pECCG117-ilvA(T381A+F383A) strain into which the ilvA(T381A+F383A) mutation was introduced was confirmed to have remarkably increased L-isoleucine production and the high L-threonine degradation rate, as compared to the KCCM11248P or KCCM11248P/pECCG117-ilvA(F383A) strain. In other words, with respect to the object of the present disclosure, it was confirmed that the ilvA (T381A+F383A) mutation was introduced to release feedback on L-isoleucine and to increase activity.

### Example 3: Preparation of Foreign gdh-introduced L-isoleucine Strains and Evaluation of Isoleucine-Producing Ability

The vector prepared in Example 1 was transformed into *Corynebacterium glutamicum* CA10-3101 prepared in Example 2 by electroporation, and through a second crossover process, a strain into which the foreign gdh was introduced on the chromosome was obtained. The introduction was confirmed through SEQ ID NO: 30 and SEQ ID NO: 31. Sequences of the primers used to perform the PCR are shown in the table below.

**[Table 5]**

| SEQ ID NO. | Name | Sequence |
|---|---|---|
| 30 | primer | AACTGATGCCTGAGGACAAG |
| 31 | primer | GCTTGATACCGAAGCAAACC |

CA10-3101ΔN2872-introduced strain was named CA10-3135, CA10-3101ΔN2872::Pn_gdh(C.gl)-introduced strain was named CA10-3136, CA10-3101ΔN2872::Pn_gdh(eco)-introduced strain was named CA10-3137, CA10-3101AN2872::Pn_rocG(B.su)-introduced strain was named CA10-3138, CA10-3101ΔN2872::Pn_gdh(rhodospirillales)-introduced strain was named CA10-3139, and CA10-3101ΔN2872::Pn_gdh(m.sm)-introduced strain was named CA10-3140.

In order to confirm the effects of increasing the L-isoleucine productivity and reducing a by-product α-aminobutyric acid (AABA) of the six types of strains thus prepared, fermentation titer was evaluated for each strain in the following manner. The parent strain and the mutant strains were inoculated into a 250 ml corner-baffled flask containing 25 ml of isoleucine production medium, respectively and then cultured with shaking at 200 rpm at 32°C for 60 hours to produce L-isoleucine.

The composition of the production medium used in this Example is as follows.

### <Production medium>

10% glucose, 0.2% yeast extract, 1.6% ammonium sulfate, 0.1% monobasic potassium phosphate, 0.1% magnesium sulfate heptahydrate, 10 mg/l ferrous sulfate heptahydrate, 10 mg/l manganese sulfate monohydrate, 200 µg/l biotin, pH 7.2

After completion of the culture, L-isoleucine and alpha-aminobutyric acid (AABA) production amounts were measured using high-performance liquid chromatography (HPLC), and the concentrations are shown in Table 6 below.

**[Table 6]**

| | L-isoleucine concentration (g/L) | AABA concentration (g/L) |
|---|---|---|
| CA10-3101 (parent strain) | 2.4 | 0.8 |
| CA10-3135 | 2.5 | 1.6 |
| CA10-3136 | 2.6 | 1.4 |
| CA10-3137 | 2.5 | 1.7 |
| CA10-3138 | 2.6 | 0.8 |
| CA10-3139 | 2.5 | 0.7 |
| CA10-3140 | 2.7 | 2.1 |

As a result, as shown in Table 6, it was confirmed that L-isoleucine was increased by 8.3% in CA10-3136 and CA10-3138, 4.2% in CA10-3135, CA10-3137, and CA10-3139, and 12.5% in CA10-3140, as compared to the parent strain. It was confirmed that the by-product AABA was increased by 100% in CA10-3135, 75% in CA10-3136, 112.5% in CA10-3137, and 162.5% in CA10-3140, as compared to the parent strain, while the by-product AABA in CA10-3138 was the same as in the parent strain, and decreased by 12.5% in CA10-3139.

Accordingly, it was confirmed that the production amount of α-aminobutyric acid (AABA) relative to the production amount of L-isoleucine was decreased in the rocG(b.su) or gdh(rhodospirillales)-introduced strain (CA10-3138, CA10-3139), as compared to the parent strain *Corynebacterium glutamicum* CA10-3101. It was also confirmed that the production amount of L-isoleucine was at the same level, but the production amount of α-aminobutyric acid (AABA) was decreased, as compared to *Corynebacterium glutamicum* ATCC13032 gdh-introduced CA10-3135. Therefore, it was confirmed that when genes encoding glutamate dehydrogenase from rocG (b.su) and gdh (rhodospirillales) among various foreign gdh genes are introduced, the by-product is reduced while L-isoleucine production is maintained, thereby improving the fermentation purity of L-isoleucine.

### Example 4: Preparation of Foreign gdh-Enhanced Strain from L-isoleucine Producing Corynebacterium glutamicum KCCM11248P strain and Evaluation

rocG (derived from *b.su)* and gdh (derived from *rhodospirillales*) which are effective in increasing the L-isoleucine-producing ability and reducing the by-product, as confirmed in Example 3, were introduced into KCJI-38 (KCCM11248P, Korean Patent No. 10-1335789) which is an L-isoleucine-producing strain treated with N-methyl-N'-nitro-N-nitrosoguanidine (NTG), by an electric pulse method, and then spread on a selection medium containing 25 mg/L of kanamycin to perform transformation. Through a second crossover process, a strain in which the foreign gdh was introduced on the chromosome was obtained. Thereafter, the concentrations of L-isoleucine and by-product in the culture medium were measured in the same manner as in Example 3, and the results are shown in Table 7 below.

**[Table 7]**

| | L-isoleucine concentration (g/L) | AABA concentration (g/L) |
|---|---|---|
| KCCM11248P (parent strain) | 1.4 | 0.7 |
| KCCM11248PΔN2872::Pn_gdh(C.gl) | 1.6 | 1.4 |
| KCCM11248PΔN2872::Pn_rocG(B.su) | 1.6 | 0.7 |
| KCCM11248P ΔN2872::Pn_gdh(rhodospirillales) | 1.7 | 0.6 |

As shown in Table 7, it was confirmed that the by-product AABA was decreased in the rocG(b.su) or gdh(rhodospirillales)-introduced strain, as compared to the parent strain KCCM11248P. Specifically, it was confirmed that the by-product AABA was decreased while the L-isoleucine productivity was maintained, and the fermentation purity of L-isoleucine was increased in the KCCM11248PΔN2872:: Pn_rocG(B.su) and KCCM11248PΔN2872::Pn_gdh(rhodospirillales) strains, unlike the KCCM11248PΔN2872::Pn_gdh(C.gl) strain in which the concentrations of L-isoleucine and AABA were all increased.

Accordingly, it was confirmed that the microorganism of the present disclosure, into which the foreign gene encoding glutamate dehydrogenase derived from *Bacillus subtilis* or *Rhodospirillales* was introduced, may produce L-isoleucine in a high yield by increasing the purity of L-isoleucine.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A microorganism with L-isoleucine-producing ability, into which a foreign gene encoding glutamate dehydrogenase derived from *Bacillus subtilis* or *Rhodospirillales* is introduced.

2. The microorganism of claim 1, wherein the foreign gene encoding glutamate dehydrogenase derived from *Bacillus subtilis;* and the foreign gene encoding glutamate dehydrogenase derived from *Rhodospirillales* have nucleotide sequences of SEQ ID NOS: 2 and 4, respectively.

3. The microorganism of claim 1, wherein the microorganism has a reduction in the production amount of α-aminobutyric acid (AABA) relative to the production amount of L-isoleucine.

4. The microorganism of claim 1, wherein the microorganism is a microorganism of the genus *Corynebacterium.*

5. The microorganism of claim 4, wherein the microorganism of the genus *Corynebacterium* is *Corynebacterium glutamicum.*

6. A method of producing L-isoleucine, the method comprising the step of culturing, in a medium, a microorganism with L-isoleucine-producing ability, into which a foreign gene encoding glutamate dehydrogenase derived from *Bacillus subtilis* or *Rhodospirillales* is introduced.

7. The method of claim 6, further comprising the step of recovering L-isoleucine from the cultured microorganism or culture medium.

8. The method of claim 6, wherein the foreign gene encoding glutamate dehydrogenase derived from *Bacillus subtilis;* and the foreign gene encoding glutamate dehydrogenase derived from *Rhodospirillales* have nucleotide sequences of SEQ ID NOS: 2 and 4, respectively.

9. The method of claim 6, wherein the method reduces the production amount of α-aminobutyric acid (AABA) relative to the production amount of L-isoleucine.

10. A composition for producing L-isoleucine, the composition comprising a microorganism with L-isoleucine-producing ability, into which a foreign gene encoding glutamate dehydrogenase derived from *Bacillus subtilis* or *Rhodospirillales* is introduced.

11. The composition of claim 10, wherein the foreign gene encoding glutamate dehydrogenase derived from *Bacillus subtilis;* and the foreign gene encoding glutamate dehydrogenase derived from *Rhodospirillales* have nucleotide sequences of SEQ ID NOS: 2 and 4, respectively.

12. The composition of claim 11, wherein the composition reduces the production amount of α-aminobutyric acid (AABA) relative to the production amount of L-isoleucine.
